(19) Europäisches Patentamt
European Patent Office
Office européen des brevets

(11) **EP 3 729 585 B1**

(12) **EUROPÄISCHE PATENTSCHRIFT**

(45) Veröffentlichungstag und Bekanntmachung des Hinweises auf die Patenterteilung:
**28.01.2026  Patentblatt 2026/05**

(21) Anmeldenummer: **18829331.0**

(22) Anmeldetag: **18.12.2018**

(51) Internationale Patentklassifikation (IPC):
*H02J 1/00* (2026.01)  *H02J 4/00* (2026.01)
*A61F 2/68* (2006.01)  *A61F 2/70* (2006.01)
*H02J 1/14* (2026.01)

(52) Gemeinsame Patentklassifikation (CPC):
**H02J 13/00002; A61F 2/68; H02J 1/14;**
A61F 2002/701; A61F 2002/702; A61F 2002/704;
H02J 1/10; H02J 7/0063; H02J 2310/23;
Y02B 70/3225; Y04S 20/222

(86) Internationale Anmeldenummer:
**PCT/EP2018/085658**

(87) Internationale Veröffentlichungsnummer:
**WO 2019/121788 (27.06.2019 Gazette 2019/26)**

(54) **VERFAHREN ZUR VERTEILUNG EINER BEGRENZTEN ELEKTRISCHEN LEISTUNG AUS EINER ENERGIEQUELLE**

METHOD FOR DISTRIBUTING A LIMITED AMOUNT OF ELECTRICAL POWER FROM AN ENERGY SOURCE

PROCÉDÉ DE DISTRIBUTION D'UNE PERFORMANCE ÉLECTRIQUE LIMITÉE PROVENANT D'UNE SOURCE D'ÉNERGIE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität:  **22.12.2017  DE 102017131194**

(43) Veröffentlichungstag der Anmeldung:
**28.10.2020  Patentblatt 2020/44**

(73) Patentinhaber: **Otto Bock Healthcare Products GmbH**
**1110 Wien (AT)**

(72) Erfinder:
• **PAPPE, Alexander**
**1180 Wien (AT)**
• **WEIGL-POLLACK, Andreas**
**3464 Goldgeben (AT)**
• **NOLTE, Michael**
**37136 Seeburg (DE)**
• **ALBRECHT-LAATSCH, Erik**
**37124 Rosdorf (DE)**
• **KAITAN, Robert**
**1220 Wien (AT)**
• **HOFFMANN, Robert**
**1160 Wien (AT)**
• **PAUSER, Thomas**
**7035 Steinbrunn (AT)**
• **SCHRAMEL, Andreas**
**1160 Wien (AT)**
• **SAGMEISTER, Luis**
**2823 Pitten (AT)**
• **EDER, Marcus**
**1090 Wien (AT)**
• **MATEJCEK, Daniel**
**2320 Mannswörth (AT)**

(74) Vertreter: **Gramm, Lins & Partner**
**Patent- und Rechtsanwälte PartGmbB**
**Frankfurter Straße 3 C**
**38122 Braunschweig (DE)**

(56) Entgegenhaltungen:
EP-A2- 3 176 896        US-A1- 2003 144 779
US-A1- 2008 052 544     US-A1- 2015 346 243

EP 3 729 585 B1

**Beschreibung**

Die Erfindung betrifft ein Verfahren zur Verteilung einer begrenzten elektrischen Leistung aus einer Energiequelle auf mehrere elektrische Verbraucher eines orthopädietechnischen Systems.

Als orthopädietechnische Komponenten werden Orthesen, Prothesen, Rollstühle, Datenlogger, Funkmodule, Feedbackelemente, elektrische Speichereinrichtungen oder Teile von Orthesen oder Prothesen oder Rollstühlen angesehen, beispielsweise Prothesengelenke, Prothesenfüße, Rohradapter, Prothesenhände, Prothesenellenbogen, Drehadapter, Prothesenschäfte, Orthesenschienen, Orthesengelenke, Fußschalen sowie daran angeordnete Sensoren, Speichereinrichtungen, Prozessoren oder andere Datenverarbeitungseinrichtungen. Ein System aus mehreren orthopädietechnischen Komponenten ist eine Kombination solcher Komponenten zu einer orthopädietechnischen Einheit, die eine über die Funktionalität der einzelnen orthopädietechnischen Komponente hinausgehende Funktionalität aufweist. Ein solches System kann beispielsweise eine Oberschenkelprothese aus einem Oberschenkelschaft, ein Prothesenkniegelenk, ein Unterschenkelrohr sowie ein Prothesenfuß sein. Das System einer Unterschenkelprothese besteht aus einem Unterschenkelschaft und einem daran angeordneten Prothesenfuß. Prothesen einer oberen Extremität als Oberarmprothese können einen Schaft, einen aktiven Ellbogen, ein Handgelenk und einen Greifer aufweisen. Ein Orthesensystem kann beispielsweise als eine Knie-Knöchel-Fuß-Orthese ausgebildet sein.

Orthopädietechnische Komponenten sind häufig mit elektrischen und/oder elektronischen Einrichtungen versehen, um beispielsweise wirksame Kräfte, Raumlagen, Momente oder Zuordnungen von Bauteilen zueinander zu detektieren. Darüber hinaus kann auf der Grundlage von ermittelten Sensorwerten, die in einem Prozessor ausgewertet werden, eine Veränderung von Widerständen einer Dämpfereinrichtung oder eine Aktivierung oder Deaktivierung eines motorischen Antriebes erfolgen. Die Nutzungsdaten der orthopädietechnischen Komponente können ausgewertet werden, beispielsweise um für zukünftige Einstellungen eine Datengrundlage zu haben. Darüber hinaus können Funktionsprüfungen durch angeschlossene Prüfeinrichtungen durchgeführt werden. Komplette prothetische oder orthetische Einrichtungen, wie computergesteuerte Armprothesen oder Exoskelette werden für aufwendige Versorgungen benötigt.

Bei komplexen orthopädietechnischen Systemen weist jeder Aktuator oder elektrische Verbraucher einen ihm zugeordneten Energiespeicher auf. Darüber hinaus können die Abstimmungen beispielsweise eines prothetischen Kniegelenkes mit einem aktuierbaren prothetischen Knöchelgelenk aufwendig sein und gegebenenfalls unabhängig voneinander durchgeführt werden. Auch bei nicht angetriebenen Komponenten können elektronische Einrichtungen vorgesehen sein, beispielsweise um Belastungen oder Winkellagen über integrierte Sensoren zu erfassen. Durch den modularen Aufbau und Prothesen oder Orthesen existiert eine Vielzahl unterschiedlicher Kombinationsmöglichkeiten, was die Komplexität bei einem Softwareabgleich oder einer Energieversorgung erhöhen kann.

Insbesondere bei einem stationären Betrieb oder bei einem Aufladen eines Energiespeichers innerhalb des orthopädietechnischen Systems kann das Problem auftauchen, dass aufgrund der Vielzahl unterschiedlichster Gerätekombinationen und bei einem steigenden Energie- und Leistungsbedarf Schwierigkeiten bei der Verteilung der zur Verfügung stehenden elektrischen Leistung auftreten, damit ein einheitliches Verhalten auch unterschiedlicher orthopädietechnischer Systeme gewährleistet ist. Insbesondere beim Laden kann die durch das Netzteil zur Verfügung stellbare Leistung nicht ausreichend sein. Entsprechende Probleme und Schwierigkeiten, wie sie anhand eines orthopädietechnischen Systems beschrieben worden sind, sind auch in anderen technischen Systemen mit elektrischen Verbrauchern vorhanden.

Die US 2008/0052544 A1 beschreibt ein tragbares elektronisches Gerät, das ein Leistungs-Management-System aufweist. Sofern die verfügbare elektrische Leistung nicht zur Versorgung aller Verbraucher ausreicht, werden einer oder mehrere der Verbraucher abgeschaltet.

Die EP 3 176 896 A2 betrifft ein Verteilersystem für elektrische Leistung, das beispielsweise in einem Flugzeug zum Einsatz kommen soll. Das System weist zumindest eine Versorgungsleitung auf, von der Verbraucher getrennt werden können.

Die US 2003/0144779 A1 beschreibt ein Energieverteilungssystem für ein Kraftfahrzeug, bei der die insgesamt benötigte Leistung in einen essentiellen Betrag und einen spezifischen Betrag unterteilt ist. Das System dient zum Einsparen von elektrischer Energie bzw. Kraftstoff. Wenn die benötigte Leistung einen vorbestimmten Grenzwert überschreitet, werden bestimmte, nicht essentielle Verbraucher abgeschaltet, um den Verbrauch zu verringern bzw. zu begrenzen. Zudem ist es möglich, in Abhängigkeit eines erkannten und einen Grenzwert überschreitenden Energieverbrauchs, bestimmte Verbraucher mit einer geringeren Energiemenge zu versorgen, um ebenfalls Energie zu sparen. Dies erfolgt jedoch nicht infolge einer zur optimalen Versorgung nicht ausreichenden Energiemenge, sondern lediglich aufgrund eines einen Grenzwert überschreitenden Verbrauchs.

Die US 2015/0346243 A1 beschreibt unter anderem eine Vorrichtung zur Ermittlung der elektrischen Leistung einer Energiequelle, an der eine Vielzahl von Verbrauchern angeschlossen ist. Für jeden der Verbraucher ist ein Widerstand vorgesehen, dessen Wert mit dem Verbrauch der jeweiligen Vorrichtung korrespondiert. Die Widerstände sind parallel zueinander angeordnet. Eine Messeinheit ist eingerichtet, um einen kombinierten Widerstandswert der parallel geschalteten Widerstände zu messen.

Aufgabe der vorliegenden Erfindung ist es daher, ein Verfahren zur Verteilung einer begrenzten elektrischen Leistung bereitzustellen, mit dem eine Überlastung einer Energiequelle oder eines Netzteils verhindert wird, ohne dass die Funktionalität des Systems gefährdet wird.

Erfindungsgemäß wird diese Aufgabe durch ein Verfahren mit den Merkmalen des Hauptanspruches gelöst. Vorteilhafte Ausgestaltungen und Weiterbildungen der Erfindung sind in den Unteransprüchen, der Beschreibung sowie den Figuren offenbart.

Das Verfahren zur Verteilung einer begrenzten elektrischen Leistung aus einer Energiequelle auf mehrere elektrische Verbraucher eines orthopädietechnischen Systems sieht vor, dass zunächst die zur Verfügung elektrische Leistung der Energiequelle erfasst und/oder kodiert wird. Ein Leistungsgleichgewicht der elektrischen Verbraucher wird durch Erfassung und/oder Kodierung der entnommenen elektrischen Leistung in den einzelnen Verbrauchern überwacht. Wird festgestellt, dass die zur Verfügung stehende elektrische Leistung nicht ausreicht, um alle Verbraucher mit der benötigten elektrischen Leistung zu versorgen, wird die entnommene Leistung in den Verbrauchern reduziert. Dazu ist es vorgesehen, dass alle Verbraucher innerhalb des Systems ihren Verbrauch reduzieren können, um zu ermöglichen, dass die Verbraucher mit einer verringerten Leistung betreibbar sind, um ihren Verbrauch zu verringern, beispielsweise auf 40% oder 50% der angegebenen Maximalleistung. Wenn die entnommene Leistung in den Verbrauchern reduziert wird, kann das gesamte orthopädietechnische System weiterbetrieben werden, wenn auch nur mit einer eingeschränkten Leistungsfähigkeit, ohne dass die Gefahr besteht, dass einzelne Funktionen überhaupt nicht mehr ausgeführt werden können. Es wird mit dem Verfahren eine einheitliche Leistungsverteilung, insbesondere eine einheitliche Verteilung elektrischer Energie auf Energiespeicher geschaffen, mit dem es möglich ist, mehrere elektrische Verbraucher aus einer Energiequelle mit elektrischer Leistung zu versorgen oder mehrere elektrische Verbraucher oder Energiespeicher über ein Netzteil mit Energie zu versorgen oder zu laden. Durch die Erfassung und Kodierung der zur Verfügung stehenden elektrischen Leistung der Energiequelle ist es möglich, verschiedene Netzteile oder Energiequellen unterschiedlicher Leistungsklassen zu verwenden, da zunächst erfasst wird, wie groß die zur Verfügung stehende elektrische Leistung der Energiequelle ist und in Abhängigkeit von der zur Verfügung stehenden elektrischen Leistung die entnommene Leistung angepasst wird. Die der Energiequelle entnommene Leistung kann dazu verwendet werden, eine Aktion in oder an dem orthopädietechnischen System auszuführen, z.B. einen Verstellvorgang über einen Motor zu veranlassen. Alternativ oder ergänzend kann die entnommene Leistung zum Aufladen eines oder mehrerer Energiespeicher verwendet werden. Eine Kodierung der zur Verfügung stehenden Leistung erfolgt über digitale Signale zwischen der Energiequelle und dem zumindest einem Verbraucher, wobei die Verbraucher an einem Datenbus angeschlossen werden, über den die an den jeweiligen Verbraucher übertragende Leistung an ein Steuergerät übertragen und ein Leistungsgleichgewicht in allen Verbrauchern errechnet werden. Die Aufteilung der verfügbaren elektrischen Leistung, die auf alle Verbraucher verteilt werden kann, wird errechnet, z.B. in einem Computer, um daraus abzuleiten, wie viel Leistung auf den jeweiligen Verbraucher verteilt werden kann. Werden eine analoge und eine digitale Kodierung gleichzeitig angewendet, ist in einer Weiterbildung der Erfindung vorgesehen, dass die digitale Kodierung priorisiert wird, da digitale Signale in einem Steuergerät leichter zu verarbeiten sind.

Die Kodierung der zur Verfügung stehenden elektrischen Leistungsenergiequelle kann über deren Spannungskennlinie oder über einen Widerstand erfolgen. Die Energiequelle kann über eine Steckverbindung oder einen Gerätestecker mit dem elektrischen Verbraucher oder dem Energiespeicher verbunden werden. Über eine Zusatzleitung innerhalb des Gerätesteckers kann über eine analoge Gleichspannung das Verhältnis der zum Aufladen oder Betreiben der elektrischen Verbraucher zur Verfügung stehenden Leistung zur benötigten Leistung ermittelt werden. Ein Spannungswert dieser Gleichspannung wird festgelegt, der allen angeschlossenen elektrischen Verbrauchern eine vollständige Auslastung der Energiequelle signalisiert. Werden niedrigere Spannungen gemessen, heißt dies, dass die Leistungsaufnahme durch die elektrischen Verbraucher reduziert werden muss. Dazu ist es vorgesehen, dass der Spannungswert von allen elektrischen Verbrauchern erfasst wird.

Die maximal benötigte Leistung eines Verbrauchers kann ebenfalls über einen elektrischen Widerstand ermittelt und kodiert werden. Über den elektrischen Widerstand und den maximalen Widerstandswert errechnet sich die maximal aufgenommene elektrische Leistung, wobei der Widerstand aus der angenommenen Leistung eine Referenzenergiequelle, einem Widerstand bei Referenzleistung, der benötigten Leistung, der zur Verfügung stehende Leistung und der verfügbaren Spannung errechnet werden kann.

Vorteilhafterweise wird die Reduzierung der entnommenen Leistung in allen Verbrauchern gleichmäßig durchgeführt, so dass alle Verbraucher gleichmäßig die entnommene Leistung reduzieren, beispielsweise alle Verbraucher um 50%. Alternativ kann in Abhängigkeit von dem Bedarf der jeweiligen Verbraucher die Reduzierung individuell erfolgen, beispielsweise indem die für die Funktion besonders wichtigen Verbraucher bevorzugt werden und eine verringerte Reduktion der entnommenen Leistung erfolgt oder indem der Ladestrom in einzelnen oder allen Verbrauchern reduziert wird. Die jeweiligen elektrischen Verbraucher können mit einer Kennung oder Kodierung versehen sein, die eine Abstufung der Reduzierung unter den mehreren elektrischen Verbrauchern bewirkt. Eine solche Kodierung kann auch dynamisch erfolgen, beispielsweise in Abhängigkeit von detektierten Belastungssituationen, bei denen eine unterschiedliche Bevorzugung oder Präferierrung einzelner Verbraucher unterschiedlich erfolgen kann.

Eine Kodierung der zur Verfügung stehenden Leistung über analoge Signale ist eine Möglichkeit, besonders einfach und robust die zur Verfügung stehende Leistung zu kodieren. Die Kodierung kann über einen Pullup-Widerstand in einem Netzteil oder eine Kodierung der maximal zu entnehmenden Leistung über zumindest einen Pull-down-Widerstand in zumindest einem Verbraucher erfolgen. Dazu sind in allen Geräten ein Pulldown-Widerstand vorgesehen, der von einer Kontrollleitung an Masse gelegt ist, wobei der Pulldown-Widerstand optional deaktiviert werden kann, wenn beispielsweise ein Energiespeicher über eine Netzteil nicht geladen wird. Die Kodierung der zur Verfügung stehenden Leistung über einen Pullup-Widerstand sieht in dem Netzteil oder in der Energiequelle einen Widerstand vor, der in einer Kontrollleitung von der Energiequelle zu dem Gerätestecker führt. Über den Pullup-Widerstand kann die zur Verfügung stehende Versorgungsleistung festgelegt werden.

Die Kodierung der zur Verfügung stehenden Leistung kann über ein optisches Medium oder drahtlos übertragen werden, so dass eine Anpassung der entnommenen Leistung auch bei einer drahtlosen Kopplung oder unabhängig von einer drahtlosen Kopplung erfolgen kann.

Eine Weiterbildung der Erfindung sieht vor, dass die Kodierung der zur Verfügung stehenden Leistung in einem Stecker erfolgt, der den und die Verbraucher mit der Energiequelle, insbesondere einem Netzteil oder einem Akku, verbindet. Über einen solchen Stecker oder eine solche Steckeraufnahme, der oder die dem orthopädietechnischen System zugeordnet ist, besteht die Möglichkeit, unabhängig von der Energiequelle, die als Netzteil oder Akku ausgebildet sein kann, festzulegen, welche Leistung wo benötigt wird und wieviel Energie zu welchem Zeitpunkt an welchen Verbraucher geleitet wird. Der Stecker oder die Steckeraufnahme kann mit einem Widerstand oder einer Platine mit einer entsprechenden Schaltung versehen sein, um die zur Verfügung stehende Leistung ebenso wie die entnommene Leistung und auch die maximal zu entnehmende Leistung detektiert, kodiert und gegebenenfalls reduziert.

Verbrauchern, insbesondere allen Verbrauchern, kann jeweils eine Kennung zugeordnet sein, wobei alle mit einer Kennung versehenen Verbraucher des Systems identifiziert und authentifiziert werden. Jedem identifizierten und authentifizierten Verbraucher kann eine ihm zugeordnete Leistungsfreigabe erteilt werden. Nur nach Authentifizierung wird überhaupt eine Leistung von einem Netzteil oder einer Energiequelle dem jeweiligen Verbraucher zugeleitet. Dadurch wird gewährleistet, dass nur genehmigte und authentifizierte elektrischer Verbraucher in dem orthopädietechnischen System einsetzbar sind und mit Energie versorgt werden. Dadurch wird die Sicherheit für einen Nutzer des orthopädietechnischen Systems erhöht, da sichergestellt wird, dass nur geprüfte orthopädietechnische Komponenten als Teil des orthopädietechnischen Systems eingesetzt werden oder eingesetzt werden können.

Als Verbraucher werden alle Bauteile oder Baugruppen angesehen, die Energie von der Energiequelle aufnehmen und speichern oder verbrauchen können.

Die Kodierung der zur Verfügung stehenden elektrischen Leistung erfolgt bevorzugt in der Energiequelle

Nachfolgend wird ein Ausführungsbeispiel der Erfindung anhand der beigefügten Figuren näher erläutert. Es zeigen:

Figur 1 - eine schematische Darstellung eines Systems mit einer Energiequelle und zwei Verbrauchern;

Figur 2 - eine Variante der Figur 1 mit zwei optionalen Energiequellen;

Figur 3 - eine Variante der Figur 1 mit einem digitalen Bus als Verbindung zwischen zwei Verbrauchern;

Figur 4 - eine Variante der Figur 3 mit unterschiedlichen Netzteilen;

Figur 5 - eine Beispieldimensionierung für einen Pullup-Widerstand in einem Netzteil;

Figur 6 - eine Beispieldimensionierung für einen Pull-down-Widerstand in Verbrauchern; sowie

Figur 7 - eine Beispielberechnung für einen Leistungsfaktor.

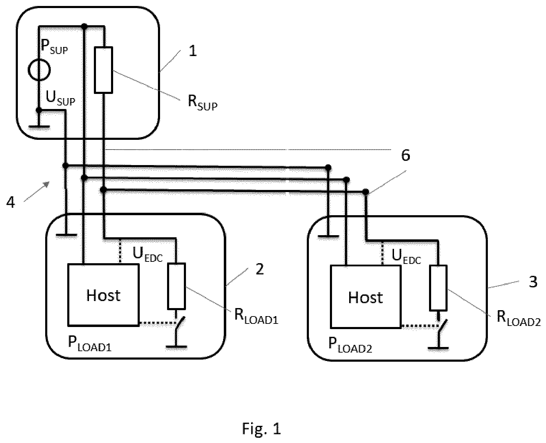

In der Figur 1 ist in einer schematischen Darstellung ein Netzteil als einzige Energiequelle 1 zur Versorgung mehrerer elektrischer Verbraucher 2 und 3 dargestellt. Das Netzteil ist über einen Gerätestecker 4 mit dem ersten Verbraucher 2 verbunden. Über eine Kabelverbindung, die ebenfalls über einen Stecker 5 realisiert werden kann, ist der zweite elektrische Verbraucher 3 ebenfalls an dem Steckernetzteil 1 angeschlossen. Die Kabelverbindung kann auch über den Gerätestecker 4 oder einen weiteren Kabelanschluss an dem Verbraucher 2 realisiert werden. Bei einer herkömmlichen Kopplung eines Netzteils 1 mit mehreren Verbrauchern 2, 3 ist es problematisch, die zum Aufladen zur Verfügung stehende Leistung $P_{SUP}$, die von dem Netzteil 1 abgegeben werden kann, zu überwachen. Aufgrund von Bauraumbeschränkungen kann es notwendig sein, nur einen Steckeranschluss 4 für ein Netzteil 1 in einem System mit mehreren Verbrauchern 2, 3 vorzusehen. Bauraumbeschränkungen gibt es beispielsweise bei orthopädietechnischen Systemen wie Orthesen, Prothesen oder Rollstühlen. Darüber hinaus können aufgrund eines modularen Aufbaus unterschiedliche Gerätekombinationen mit unterschiedlichen Verbrauchern oder Energiespeichern bereitzustellen sein, beispielsweise um durch Einbau unterschiedlicher Verbraucher eine Anpassung an den jeweiligen Kundenwunsch zu ermöglichen oder um eine Anpassung an einen Anwender vorzunehmen. Darüber hinaus kann bei Weiterentwicklungen oder bei einem Modulaustausch ein steigender Energie- und Leistungsbedarf auftreten, der bei der ursprünglichen Auslegung nicht absehbar war. Ebenso können verschiedene elektrische Energiequellen oder Netzteile mit unterschiedlichen Leistungsklassen vorhanden sein, die für ein System einsetzbar sind. Bei einer starren Festlegung von zur Verfügung stehender oder maximal abzugebender Leistung kann mit einem herkömmlichen Netzteil und mit herkömmlichen Verbrauchern, die über zwei Leiter mit einer Gleichspannung versorgt werden, nicht möglich. Es ist wünschenswert, ein System bereitzustellen, das eine Flexibilität gegenüber Veränderungen sowohl in der Hardware als auch hinsichtlich der Einsatzbedingungen zulässt. Daher ist es erfindungsgemäß vorgesehen, dass neben Masse und Leiter zum positiven Pol eine Steuerleitung 6 sowohl in dem Netzteil 1 oder der Energieversorgung 1 als auch in den Verbrauchern 2, 3 vorgesehen ist, über die eine Kodierung der Leistung erfolgen kann. Die Steuerleitung 6 oder auch Energieverteilleitung wird an alle Steckeranschlüsse 4, 5 geführt. Die Steuerleitung 6 zeigt über eine analoge Gleichspannung $U_{EDC}$ das Verhältnis der zum Aufladen zur Verfügung stehenden Leistung $P_{SUP}$ zu der jeweils benötigten Leistung $P_{LOAD1}$, $P_{LOAD2}$ an. Der Spannungswert $U_{EDC}$ ist also der Quotient aus der zum Aufladen zur Verfügung stehenden Leistung $P_{SUP}$ und der jeweils an dem Verbraucher 2, 3, benötigten Leistung $P_{LOAD}$.

Damit für mögliche Erweiterungen oder Veränderungen in dem System mit zusätzlichen elektrischen Verbrauchern oder mit veränderten elektrischen Verbrauchern oder aber bei einer Kombination mit anderen Netzteilen die Funktionsfähigkeit weiter gewährleistet ist, ist zur Auslegung des Systems zunächst vorteilhaft, dass ein Standardnetzteil oder eine Standardenergiequelle mit einem dazugehörigen Pullup-Widerstand festgelegt wird. Alle weiteren und zukünftigen Netzteile 1 müssen zu diesem Standardnetzteil kompatibel ausgelegt sein.

Weiterhin ist es vorteilhaft, dass ein Spannungswert für die Gleichspannung $U_{EDC}$ festgelegt wird, bei dem für alle angeschlossenen Geräte eine vollständige Auslastung des jeweils angeschlossenen Netzteils 1 signalisiert wird. Werden niedrigere Spannungen $U_{EDC}$ ermittelt, heißt dies, dass die jeweilige Leistungsaufnahme in den Verbrauchern 2, 3 reduziert werden muss. Der Wert für die Spannung $U_{EDC}$ ist in allen angeschlossenen Verbrauchern 2, 3 messbar.

Zunächst wird die Versorgungsspannung für die Energieverteilungssteuerung mit einer vereinheitlichten Ladespannung definiert. Sollten in Zukunft veränderte Ladespannungen benötigt werden, muss dies netzteilseitig auf das jeweils definierte Niveau angepasst werden.

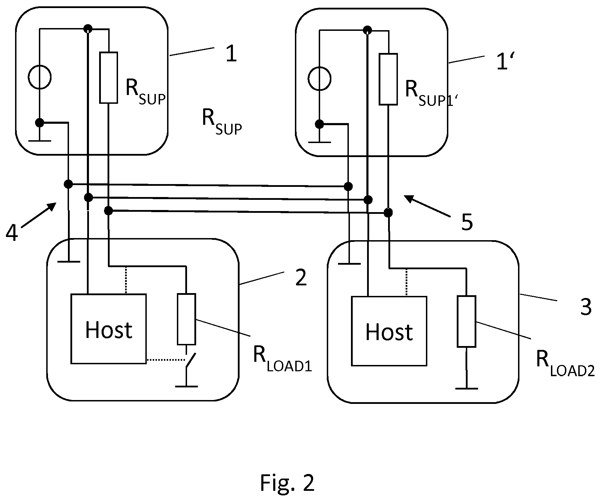

In der Figur 2 ist eine Variante der Figur 1 dargestellt, bei der zwei unterschiedliche Netzteile 1, 1' mit unterschiedlichen Pullup-Widerständen für unterschiedliche Leistungen vorhanden sind. Das Netzteil 1 hat den Pullup-Widerstand $R_{SUP1}$, das zweite Netzteil 1' weist einen abweichenden Pullup-Widerstand $R_{SUP1'}$ auf, der beispielsweise 50% höher als der Pullup-Widerstand $R_{SUP}$ liegen kann.

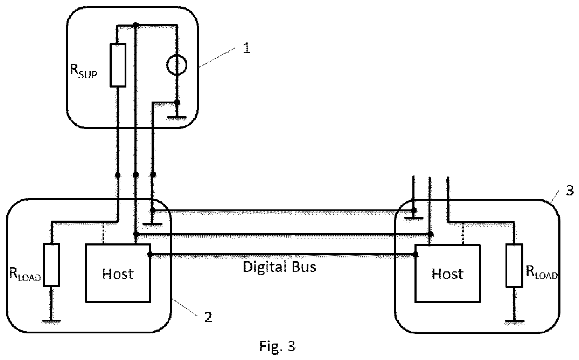

Bei einem Ausführungsbeispiel gemäß Figur 3 erfolgt die Codierung der zur Verfügung stehenden Leistung über digitale Signale mittels eines Signalbusses, analog zu dem Ausführungsbeispielen gemäß der Figuren 1 und 2, jedoch nur zu einem Verbraucher 2. Zusätzlich findest ein Signalaustausch zwischen den Verbrauchern 2, 3 statt. Der Signalbus ist zwischen den Verbrauchern 2, 3 installiert. Der erste Verbraucher 2 ermittelt, wieviel Energie auf die nachfolgenden Verbraucher 3 verteilt wird und übernimmt die Verteilung der Energie innerhalb des Systems, beispielsweise einer Orthese oder Prothese. Dafür benutzt er den Signalbus. Die Pulldown-Widerstände $R_{LOAD}$ in den Verbrauchern sind in diesem Ausführungsbeispiel gleich.

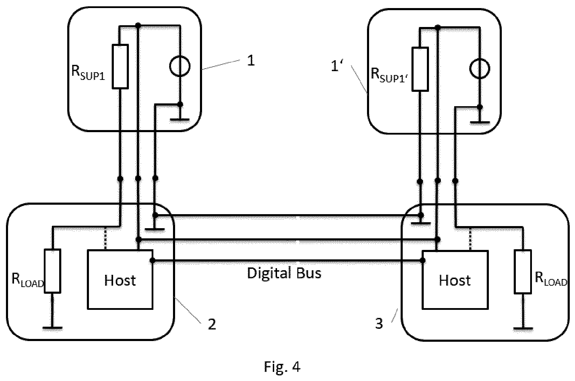

In der Figur 4 sind zwei Netzteile 1, 1' an jeweils an einen Anschluss 4 des jeweiligen Verbrauchers 2, 3 angeschlossen. Es sind beide Netzteile 1, 1' angesteckt, erfolgt ein interner Abgleich zwischen den Verbrauchern 2, 3, wie die Energie aus den Netzteilen 1, 1' auf die jeweiligen Verbraucher verteilt werden soll.

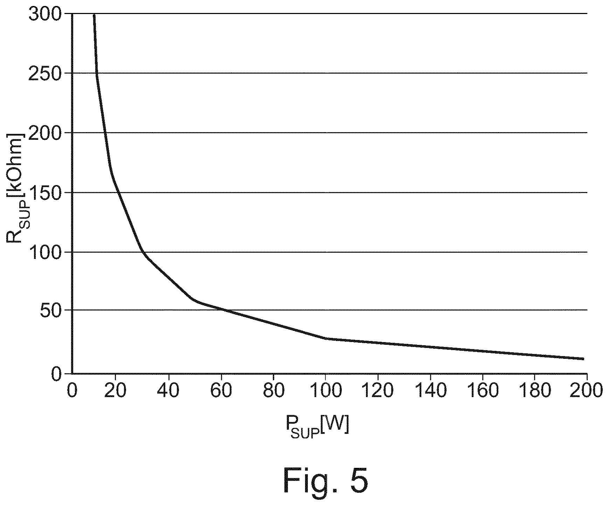

In der Figur 5 ist eine beispielhafte Dimensionierung der Widerstände in Abhängigkeit von der zur Verfügung stehenden Leistung $P_{SUP}$ dargestellt. Alle Netzteile 1, 1' müssen einen Pullup-Widerstand $R_{SUP}$ von der Versorgungsspannung $U_{SUP}$, die auf der Steuerleitung 6 anliegt, implementieren, um darüber die verfügbare elektrische Leistung $P_{SUP}$ zu kodieren. Der notwendige minimale Wert des jeweiligen Pullup-Widerstandes errechnet sich aus

$$R_{sup\,min} = R_{Ref}\frac{P_{Ref}}{P_{Sup}}$$, wobei $P_{Ref}$ die Leistung eines Referenznetzteils ist und $R_{Ref}$ der Pullup-Widerstand bei einer Referenzleistung. Ein möglicher Verlauf des Verhältnisses von zur Verfügung stehender Leistung $P_{SUP}$ zum minimalen Pullup-Widerstand ist in dem Diagramm der Figur 5 aufgezeichnet.

Alle Verbraucher 2, 3 müssen ein Pulldown-Widerstand $R_{LOAD}$ von der Steuerleitung 6 zur Masse implementieren, um ihre jeweils maximal aufgenommene elektrische Leistung zu kodieren. Sollte der Verbraucher gerade nicht mit Energie versorgt werden, kann der Pulldown-Widerstand $R_{LOAD}$ deaktiviert werden, was durch den Schalter in der Figur 1 und der Figur 2 angedeutet ist.

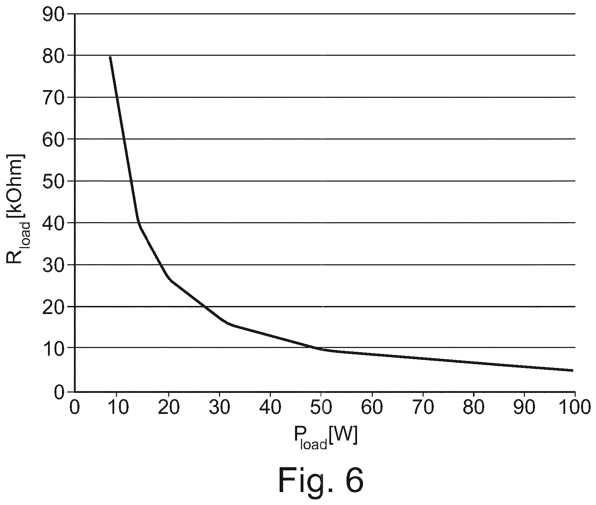

Der notwendige maximale Widerstandswert für den jeweiligen Pulldown-Widerstand $R_{LOAD}$ errechnet sich aus $\frac{R_{Ref}\, P_{Ref}}{P_{LOAD}} \; x \; \frac{U_{100\%}}{U_{SUP} - U_{100\%}}$ . Ein beispielhafter Kurvenverlauf des Verhältnisses zwischen der benötigten Leistung $P_{LOAD}$ und dem Pulldown $R_{LOAD}$ ist in dem Diagramm der Figur 6 gezeigt.

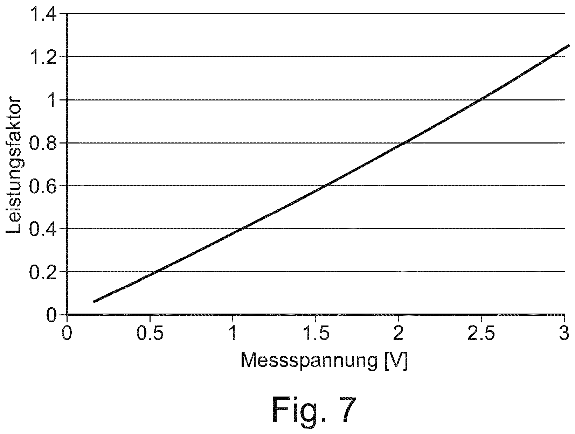

Alle Verbraucher 2, 3 müssen die Kontrollspannung $U_{EDC}$ auswerten. Wird die Referenzspannung $U_{100\%}$ nicht erreicht, ist die zur Verfügung stehende elektrische Leistung $P_{SUP}$ kleiner als die benötigte Leistung, so dass die jeweils aufgenommene Leistung in den Verbrauchern 2, 3 reduziert werden muss. Die verfügbare relative Leistung k ergibt sich aus dem Quotienten der zur Verfügung stehenden Leistung $P_{SUP}$ durch die Summe der benötigten Leistungen $P_{LOAD}$. Liegt die relative Leistung über 1, ist genügend Leistung von dem Netzteil 1 oder den Netzteilen 1 verfügbar. Liegt der Wert für die verfügbare relative Leistung k unter 1, ist zu wenig Leistung verfügbar. Ein Verbraucher darf maximal die ihm anteilig zur Verfügung stehende Leistung $P_{max}$ aufnehmen, die maximal zur Verfügung stehende Leistung $P_{max}$ errechnet sich aus dem Produkt der verfügbaren relativen Leistung k mit der benötigten Leistung $P_{LOAD}$. Das Verhältnis von Messspannung $U_{EDC}$ zum Leistungsfaktor k ist in der Figur 7 dargestellt. In dem Berechnungsbeispiel liegt der Schwellwert für die Messspannung $U_{EDC}$ bei 2,5V, um sicherzustellen, dass alle Verbraucher 2, 3 mit ausreichend Energie versorgt werden.

An dem jeweiligen Netzteil 1, 1' kann eine Statusanzeige über die verfügbare Leistung angebracht werden. Dazu kann eine Codierung beispielsweise in drei Stufen festgelegt werden, die den Leistungsfaktoren entsprechen. Oberhalb des Leistungsfaktors 1 kann eine grüne Statusanzeige signalisieren, dass ausreichend Leistung verfügbar ist, unterhalb des Leistungsfaktors von 0,5 eine rote Statusanzeige eine unzureichende Leistung signalisieren, dazwischen kann beispielsweise über eine gelbe Leistungsanzeige signalisiert werden, dass der Ladevorgang verlängert wird.

## Patentansprüche

1. Verfahren zur Verteilung einer begrenzten elektrischen Leistung aus einer Energiequelle (1, 1') auf mehrere elektrische Verbraucher (2, 3) eines orthopädietechnischen Systems, mit den Schritten:

   a. Erfassung und/oder Kodierung der zur Verfügung stehenden elektrischen Leistung ($P_{SUP}$) der Energiequelle (1, 1'),
   b. Überwachung eines Leistungsgleichgewichtes der Verbraucher (2, 3) durch Erfassung und/oder Kodierung der entnommenen Leistung in den einzelnen Verbrauchern (2, 3), **gekennzeichnet durch**
c. Reduzierung der entnommenen Leistung in den Verbrauchern (2, 3), um die Verbraucher (2, 3) mit verringerter Leistung zu betreiben, wenn die zu Verfügung stehende Leistung ($P_{SUP}$) nicht ausreicht, um alle Verbraucher (2, 3) mit der benötigten Leistung zu versorgen, wobei die Verbraucher (2, 3) an einem Datenbus angeschlossen werden und eine Aufteilung der zur Verfügung stehenden elektrischen Leistung ($P_{SUP}$) auf alle Verbraucher (2, 3) errechnet wird und wobei die Kodierung der zur Verfügung stehenden Leistung ($P_{SUP}$) über digitale Signale zwischen der Energiequelle (1, 1') und dem zumindest einem Verbraucher (2, 3) erfolgt.

2. Verfahren nach Anspruch 1, **dadurch gekennzeichnet, dass** die Kodierung der zur Verfügung stehenden elektrischen Leistung ($P_{SUP}$) der Energiequelle (1, 1') über deren Spannungskennlinie, einen Widerstand ($R_{LOAD}$, $R_{SUP}$) oder ein analoges Spannungssignal ($U_{EDC}$) erfolgt.

3. Verfahren nach Anspruch 1 oder 2, **dadurch gekennzeichnet, dass** die maximal benötigte Leistung ($P_{LOAD}$) eines Verbrauchers (2, 3) über einen elektrischen Widerstand ($R_{LOAD}$) ermittelt und kodiert wird.

4. Verfahren nach einem der voranstehenden Ansprüche, **dadurch gekennzeichnet, dass** die Reduzierung in allen Verbrauchern (2, 3) gleichmäßig oder in Abhängigkeit von dem Bedarf individuell an den jeweiligen Verbraucher (2, 3) angepasst erfolgt.

5. Verfahren nach einem der voranstehenden Ansprüche, **dadurch gekennzeichnet, dass** die Kodierung der zur Verfügung stehenden Leistung ($P_{SUP}$) über analoge Signale erfolgt.

6. Verfahren nach Anspruch 5, **dadurch gekennzeichnet, dass** die Kodierung der zur Verfügung stehenden Leistung ($P_{SUP}$) über einen Pullup-Widerstand ($R_{SUP}$) in einem Netzteil (1, 1') und eine Kodierung der maximal zu entnehmenden Leistung über zumindest einen Pulldown-Widerstand ($R_{LOAD}$) in zumindest einem Verbraucher (2, 3) erfolgt.

7. Verfahren nach Anspruch 1, **dadurch gekennzeichnet, dass** bei einer analogen und digitalen Kodierung die digitale Kodierung priorisiert wird.

8. Verfahren nach einem der voranstehenden Ansprüche, **dadurch gekennzeichnet, dass** die Kodierung der zur Verfügung stehende Leistung ($P_{SUP}$) über ein optisches Medium oder drahtlos übertragen wird.

**9.** Verfahren nach einem der voranstehenden Ansprüche, **dadurch gekennzeichnet, dass** die Kodierung der zur Verfügung stehenden Leistung ($P_{SUP}$) in einem Stecker (4, 5) erfolgt, der den oder die Verbraucher (2, 3) mit der Energiequelle (1, 1') verbindet.

**10.** Verfahren nach einem der voranstehenden Ansprüche, **dadurch gekennzeichnet, dass** den Verbrauchern (2, 3) jeweils eine Kennung zugeordnet wird und alle mit einer Kennung versehenen Verbraucher (2, 3) des Systems identifiziert und authentifiziert werden.

**11.** Verfahren nach Anspruch 10, **dadurch gekennzeichnet, dass** jedem identifizierten und authentifizierten Verbraucher (2, 3) eine ihm zugeordnete Leistungsfreigabe erteilt wird.

**12.** Verfahren nach einem der voranstehenden Ansprüche, **dadurch gekennzeichnet, dass** die Kodierung der zur Verfügung stehenden Leistung ($P_{SUP}$) in der Energiequelle (1, 1') erfolgt.

**Claims**

**1.** A method for distributing a limited amount of electrical power from an energy source (1, 1') among a plurality of loads (2, 3) of an orthopaedic system, having the following steps:

a. measuring and/or coding the available electrical power ($P_{SUP}$) of the energy source (1, 1'),
b. monitoring a power balance of the loads (2, 3) by measuring and/or coding the drawn power in the individual loads (2, 3), **characterized by**
c. reducing the drawn power in the loads (2, 3) to operate the loads (2,3) at reduced power, if the available power ($P_{SUP}$) is not sufficient to supply all loads (2, 3) with the required power, wherein the loads (2, 3) are connected to a data bus and a division of the available electrical power ($P_{SUP}$) among all loads (2, 3) is calculated and wherein the available power ($P_{SUP}$) is coded via digital signals between the energy source (1, 1') and the at least one load (2, 3).

**2.** The method as claimed in claim 1, **characterized in that** the available electrical power ($P_{SUP}$) of the energy source (1, 1') is coded via its voltage characteristic, a resistance ($R_{LOAD}$, $R_{SUP}$) or an analog voltage signal ($U_{EDC}$).

**3.** The method as claimed in claim 1 or 2, **characterized in that** the maximum required power ($P_{LOAD}$) of a load (2, 3) is determined and coded via an electrical resistance ($R_{LOAD}$).

**4.** The method as claimed in one of the preceding claims, **characterized in that** the reduction is performed uniformly in all loads (2, 3) or is adapted individually to the respective load (2, 3) depending on the requirement.

**5.** The method as claimed in one of the preceding claims, **characterized in that** the available power ($P_{SUP}$) is coded via analog signals.

**6.** The method as claimed in claim 5, **characterized in that** the available power ($P_{SUP}$) is coded via a pull-up resistance ($R_{SUP}$) in a power supply unit (1, 1') and the maximum power to be drawn is coded via at least one pull-down resistance ($R_{LOAD}$) in at least one load (2, 3).

**7.** The method as claimed in claim 1, **characterized in that**, if an analog and digital coding are applied, the digital coding is prioritized.

**8.** The method as claimed in one of the preceding claims, **characterized in that** the available power ($P_{SUP}$) is coded via an optical medium or wirelessly.

**9.** The method as claimed in one of the preceding claims, **characterized in that** the available power ($P_{SUP}$) is coded in a plug-in connector (4, 5) which connects the load or loads (2, 3) to the energy source (1, 1').

**10.** The method as claimed in one of the preceding claims, **characterized in that** an identifier is assigned in each case to the loads (2, 3) and all loads (2, 3) of the system provided with an identifier are identified and authenticated.

**11.** The method as claimed in claim 10, **characterized in that** each identified and authenticated load (2, 3) is granted a power release which is assigned to it.

**12.** The method as claimed in one of the preceding claims, **characterized in that** the available power ($P_{SUP}$) is coded in the energy source (1, 1').

**Revendications**

**1.** Procédé de distribution d'une puissance électrique limitée, provenant d'une source d'énergie (1, 1'), entre plusieurs consommateurs électriques (2, 3) d'un système orthopédique, comprenant les étapes suivantes :

a. la détection et/ou le codage de la puissance électrique disponible ($P_{SUP}$) de la source d'énergie (1, 1'),

b. la surveillance d'un équilibre de puissance des consommateurs (2, 3) par la détection et/ou le codage de la puissance prélevée dans les différents consommateurs (2, 3),

**caractérisé par**

c. la réduction de la puissance prélevée dans les consommateurs (2, 3) afin de faire fonctionner les consommateurs (2, 3) à puissance réduite lorsque la puissance disponible ($P_{SUP}$) n'est pas suffisante pour alimenter tous les consommateurs (2, 3) avec la puissance requise,

sachant que

les consommateurs (2, 3) sont raccordés à un bus de données, et une distribution de la puissance électrique disponible ($P_{SUP}$) entre tous les consommateurs (2, 3) est calculée, et le codage de la puissance disponible ($P_{SUP}$) s'effectue via des signaux numériques entre la source d'énergie (1, 1') et ledit au moins un consommateur (2, 3).

2. Procédé selon la revendication 1,
**caractérisé en ce que** le codage de la puissance électrique disponible ($P_{SUP}$) de la source d'énergie (1, 1') s'effectue via sa courbe caractéristique de tension, via une résistance ($R_{LOAD}$, $R_{SUP}$) ou via un signal de tension analogique ($U_{EDC}$).

3. Procédé selon la revendication 1 ou 2,
**caractérisé en ce que** la puissance maximale requise ($P_{LOAD}$) d'un consommateur (2, 3) est déterminée et codée via une résistance électrique ($R_{LOAD}$).

4. Procédé selon l'une des revendications précédentes,
**caractérisé en ce que** la réduction dans tous les consommateurs (2, 3) s'effectue de manière uniforme ou de manière adaptée en fonction des besoins individuels de chaque consommateur (2, 3).

5. Procédé selon l'une des revendications précédentes,
**caractérisé en ce que** le codage de la puissance disponible ($P_{SUP}$) s'effectue via des signaux analogiques.

6. Procédé selon la revendication 5,
**caractérisé en ce que** le codage de la puissance disponible ($P_{SUP}$) s'effectue via une résistance de rappel (pull-up) ($R_{SUP}$) dans un bloc d'alimentation (1, 1'), et un codage de la puissance maximale à prélever s'effectue via au moins une résistance de tirage (pull-down) ($R_{LOAD}$) dans au moins un consommateur (2, 3).

7. Procédé selon la revendication 1,
**caractérisé en ce que**, dans le cas d'un codage analogique et numérique, le codage numérique est prioritaire.

8. Procédé selon l'une des revendications précédentes,
**caractérisé en ce que** le codage de la puissance disponible ($P_{SUP}$) est transmis via un support optique ou sans fil.

9. Procédé selon l'une des revendications précédentes,
**caractérisé en ce que** le codage de la puissance disponible ($P_{SUP}$) s'effectue dans une fiche (4, 5) qui relie le ou les consommateurs (2, 3) à la source d'énergie (1, 1').

10. Procédé selon l'une des revendications précédentes,
**caractérisé en ce qu'**un identifiant est attribué à chacun des consommateurs (2, 3), et tous les consommateurs (2, 3) du système pourvus d'un identifiant sont identifiés et authentifiés.

11. Procédé selon la revendication 10,
**caractérisé en ce que** chaque consommateur (2, 3) identifié et authentifié se voit adressé d'une libération de puissance qui lui est attribuée.

12. Procédé selon l'une des revendications précédentes,
**caractérisé en ce que** le codage de la puissance disponible ($P_{SUP}$) s'effectue dans la source d'énergie (1, 1').

Fig. 1

Fig. 2

Fig. 3

Fig. 4

Fig. 5

Fig. 6

Fig. 7

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 20080052544 A1
- EP 3176896 A2
- US 20030144779 A1
- US 20150346243 A1